# NEW EUROPEAN PATENT SPECIFICATION

(11) **EP 1 509 300 B2**
(45) Date of publication and mention of the opposition decision: **24.02.2010**
(45) Mention of the grant of the patent: 11.10.2006
(21) Application number: 03722816.0
(22) Date of filing: 08.05.2003
(51) Int. Cl.: B01D 9/00

(54) **PRODUCTION OF CRYSTALLINE MATERIALS BY USING HIGH INTENSITY ULTRASOUND**
HERSTELLUNG VON KRISTALLMATERIALIEN MIT HOCHLEISTUNGSULTRASCHALL
PRODUCTION DE SUBSTANCES CRISTALLINES A L'AIDE D'ULTRASONS A FORTE INTENSITE

(30) Priority: 31.05.2002 GB 0212626
(43) Date of publication of application: 02.03.2005
(73) Proprietor: Prosonix Limited, London EC2Y 5AB (GB)
(72) Inventor: McCausland, Linda Jane, Oxfordshire OX14 5PR (GB)
(74) Representative: Mansfield, Peter Turquand
(86) International application number: PCT/GB2003/001905
(87) International publication number: WO 2003/101577

(56) References cited:
- EP-A- 1 256 558
- WO-A-00/35579
- WO-A-00/79095
- ' Chrystallisation' CHEMICAL ENGINEERING PROGRESS September 1977, pages 34 - 43
- CONFERENCE PROCEEDINGS OF THE THIRD INTERNATIONAL CONFERENCE ON "THE SCALE-UP OF CHEMICAL PROCESSES" 21 September 1998 - 24 September 1998, JERSEY, CHANNEL ISLANDS,

## Description

This invention relates to a method for crystallisation of ingredients that may be suitable for use in pharmaceuticals.

The use of high intensity ultrasound to trigger nucleation in a supersaturated solution, so that crystallisation occurs, is known, and an apparatus for this purpose is for example described in GB 2 276 567 A. The benefits of triggering nucleation in this fashion are of particular relevance when very pure crystalline products are to be formed, as the purity of the solution and the cleanliness of the vessel surfaces means that crystallisation nuclei are not otherwise present.
Certain compounds would be desirable for use in pharmaceuticals, but have been found particularly difficult to crystallise; this relates in particular to disaccharides such as D-glucose or D-xylose. Similar problems arise with other organic compounds such as aspartic acid, and the compound α-L-aspartyl-L-phenylalanine methyl ester (aspartame). It has often been found necessary to add crystal modifiers to a saturated solution of such compounds to encourage the formation of crystals, as a saturated solution may have to be cooled considerably below the saturation temperature before crystallisation occurs; with some organic materials this under-cooling may be as much as 100 K. That is to say, a supersaturated solution may remain in a metastable state for a prolonged period, which may be many months. The use of an immersed ultrasonic probe or horn to subject a saturated solution to ultrasound is commonly used, but it has been found that some cavitation occurs at the surface of the horn, this causing erosion of the horn and consequential generation of very small metal particles (say about 0.1 mm in diameter); consequently this process would not be acceptable for generating crystalline material for use as a pharmaceutical ingredient.
Chemical Engineering Progress (Sept. 1997) (C J. Price) pp. 34-43 describes processes for improving crystallisation, and mention use of ultrasound to initiate nucleation. Conference on the Scale-Up of chemical Processes (21-24 Sept. 1998) (Scientific Update), presentation by P. Martin, mentions sononucleation achieved with ultrasound as a 2 s boost.

Accordingly the present invention provides a method for production of crystalline material, according to claim 1.

Gradual cooling of the solution in step (c) of claim 1 subsequent to the application of ultrasound, will lead to growth of the crystals formed during the ultrasonic insonation. Hence this method enables large crystals to be grown.

The ultrasound is applied to the supersaturated solution in a vessel using a multiplicity of ultrasonic transducers attached to a wall of the vessel in an array extending both circumferentially and longitudinally, each transducer being connected to a signal generator so that the transducer radiates no more than 3 W/cm², the transducers being sufficiently close together and the number of transducers being sufficiently high that the power dissipation within the vessel is between 25 and 150 W/litre. The values of power given here are those of the electrical power delivered to the transducers, as this is relatively easy to determine. Such an irradiation vessel is described in WO 00/35579. Surprisingly it has been found that with such a vessel there is no cavitation at the surface of the wall, so that there is no erosion of the wall and consequently no formation of small particles of metal. The crystalline material made by this method can be very pure, as additives are not required and the crystallisation procedure does not introduce contaminants, so that it would be suitable both for food use and for pharmaceutical use.

It is desirable to ensure no focusing of the ultrasound occurs, and this may be achieved by energising groups of adjacent transducers in succession. Where the vessel is cylindrical it is particularly preferable to avoid energising diametrically opposite transducers at the same time. The non-focusing can also be achieved by energising adjacent transducers, or adjacent groups of transducers, at different frequencies; and in particular to vary the frequency at which each transducer or group of transducers is energized over a limited range, for example between 19.5 kHz and 20.5 kHz.

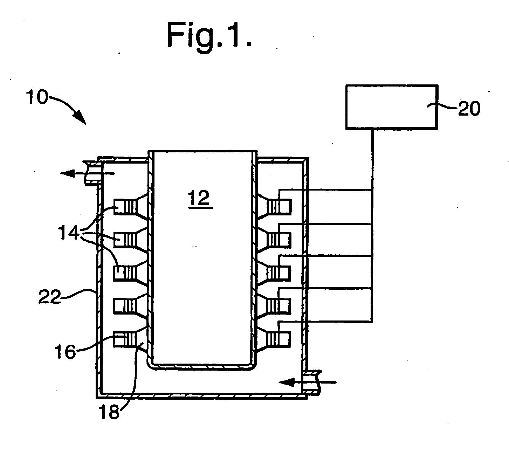

The invention will now be further and more particularly described, by way of example only, and with reference to the accompanying drawing which shows a cross-sectional view of a batch crystallisation irradiator.

Referring to the drawing, a batch crystallisation irradiator 10 includes a stainless-steel vessel 12 of internal diameter 0.31 m and of wall thickness 2 mm. To the outside of the wall are attached sixty transducer modules 14 closely packed in a square array. Each transducer module 14 comprises a 50 W piezoelectric transducer 16 which resonates at 20 kHz, attached to a conically flared titanium coupling block 18 by which it is connected to the wall, the wider end of each block being of diameter 63 mm. The transducer modules define five circumferential rings each of twelve modules 14, the centres of the coupling blocks 18 being on a square pitch of 82 mm. The irradiator 10 also incorporates three signal generators 20 (only one is shown) each of which drives the transducers 16 in a pair of adjacent longitudinal rows and another such pair of rows one third of the circumference apart from the first pair.

In use of the irradiator 10 the vessel 12 is filled with a solution and the temperature of the vessel is gradually lowered (assuming the solubility decreases as the temperature decreases) using a cooling jacket 22, and the contents of the vessel 12 are stirred. Consequently the solution will become saturated and then supersaturated. When the temperature is about 10 K below that at which saturation occurs, the transducers are energized briefly, each generator 20 being energized for 0.8 second successively. Each transducer irradiates 50 W over a circle of diameter 63 mm, that is an intensity of 1.6 W/cm². The ultrasonic energy is dissipated over the cylindrical volume of the vessel 12, which is about 31 litres, so if all the transducers 16 were energised simultaneously the power density would be about 100 W/litre. To avoid focusing, only one signal generator 20 is energized at any one time, so the energy deposition is about 33 W/litre. After 0.8 second, a different generator 20 is energized, and so on. After 2.4 seconds each transducer has been energized, and application of ultrasound is terminated. The contents of the vessel 12 are then inspected, to see if any crystals have formed. If there are no crystals this activation procedure is repeated. Once crystals are observed, application of ultrasound is terminated, and the temperature of the vessel 12 is gradually lowered.

In a modification the signal generators 20 may generate signals at a frequency that varies between 19.5 and 20.5 kHz, the signals from different signal generators 20 varying independently of each other.

With this irradiator 10 the power intensity is such that cavitation does not occur at the surface of the wall, so erosion of the vessel 12 does not occur. Nevertheless the power density is sufficient to ensure nucleation in a saturated solution.

An experiment to investigate the effect of ultrasound on crystallisation has been performed, as follows. An aqueous solution of D-xylose containing 25 g D-xylose per 10 ml water was prepared, which would be saturated at 50°C. This was than cooled at a rate of 0.2 K/min to 20°C, and the resulting solid products were separated and isolated. As a control, in one case the transducers 14 were not energized; in this case crystals did not appear until the temperature had dropped to 36°C. If the transducers 14 were energized for a period of 2 minutes, starting at 46°C, then crystals appeared at 43°C. If the transducers 14 were energized continuously, starting at 50°C, then the resulting crystals were very small, and information on sizes was not obtained. Table 1 gives the temperature T at which solid first appeared and also shows the effect on crystal size distribution by indicating the crystal size (in µm) for different cumulative percentiles (by mass):

**Table 1**

| Conditions | T/°C | 10% | 50% | 90% |
|---|---|---|---|---|
| No ultrasound | 36 | 27 | 67 | 149 |
| 2 min ultrasound | 43 | 43 | 106 | 211 |
| Ultrasound | 46 | - | - | - |

Since the solutions were saturated at 50°C, ideally crystallisation should commence as soon as the temperature drops below 50°C. The short application of ultrasound markedly reduces the metastable zone width to only about 7 K (as compared to about 14 K in the absence of ultrasound). It also gives a significant increase in the crystal sizes that are formed. Continuous application of ultrasound reduces the metastable zone width even more, to about 4 K.

It will be appreciated that the conditions that applied in this particular experiment do not exactly correspond to the method of the present invention, but that the results indicate that it would be appropriate to cool the solution to about 43°C before subjecting it to brief irradiation.

In performing the present invention; the temperature to which the solution is to be cooled before the brief application of ultrasound will differ for different solutions, depending on the material, the solvent and the concentration, and must therefore be found by experiment. It may be ascertained by experiments similar to those described above. The solution is first subjected to continuous ultrasound as it is cooled, and the temperature at which crystals form (T, which in the example above was 46°C) is observed. Further tests are then carried out, cooling the solution to different temperatures within a few degrees above or below T to find the highest temperature at which crystals form on application of a brief pulse of ultrasound. Typically this is within 5 K of the temperature T observed with continuous ultrasound.

Aspartame is the α-dipeptide ester L-aspartyl-L-phenylalanine methyl ester and is an important synthetic low-calorie sweetening agent. It is about 200 times sweeter than sugar and does not leave a bitter aftertaste, and so is used in a wide range of products. It is, however, difficult to crystallise without use of crystal modifiers, particularly from aqueous solution. Surprisingly, it has been found possible to produce satisfactory crystals of aspartame directly from an aqueous solution using the present method. A saturated solution of aspartame in warm pure water is prepared, and introduced into the vessel 12. The temperature of the solution is gradually cooled to about 10 K below the temperature at which it would be saturated, and is subjected to ultrasonic irradiation as described above for a short time, for example 2.4 s. The solution is then inspected, and if crystals have formed as a result of the ultrasonic irradiation, then the temperature of the vessel is gradually cooled over a period of a few hours down to room temperature.

This process has been found to produce aspartame crystals between 100 and 250 µm in size, which are easy to separate from the remaining liquid for example by filtration. By avoiding the need for additives the purity of the product is ensured.

The inspection to check if any crystals have formed as a result of the ultrasonic irradiation may be an inspection by eye, while shining a light into the solution, as the small crystals sparkle.

It will be appreciated that the method is applicable using different apparatus, and may be applied on a continuous rather than a batch basis. For example a saturated solution may be caused to flow along a duct in which its temperature gradually decreases, the duct incorporating a flow-through ultrasonic irradiation module at a position at which the solution has reached the appropriate temperature, so that the solution is briefly irradiated as it flows through the module. In this case the transducers of the ultrasonic irradiation module might be activated continuously or in a pulsed mode.

The method is applicable to many different chemical compounds. For example it may be used for proteins and amino acids, and for antibiotics. By way of example, the following measurements have been made with the three amino acids L-leucine, L-phenylalanine, and L-histadine.

Saturated solutions in water were prepared at 75°C, the concentrations being 3.3, 6.2 and 11.3 g/100 g water respectively (after 24 hours in contact with solid material). Four samples were taken of each solution, and were then cooled at a steady rate of 0.2°C/min. In half the cases the samples were subjected to a 10 s burst of ultrasound every 5 minutes until crystals were observed. No ultrasound was applied to the others. The temperature, T, at which crystals first appeared is shown in Table 2, Tu being the cases with ultrasound, and Tx those without ultrasound.

| TABLE 2 | Tx/°C | Tx/°C | Tu/°C | Tu/°C |
|---|---|---|---|---|
| L-leucine | 52.9 | 52.2 | 66.0 | 64.5 |
| L-phenylalinine | 65.2 | 66.4 | 69.1 | 71.0 |
| L-histadine | 65.5 | 67.0 | 69.0 | 70.1 |

It will be appreciated that in each case the application of ultrasound reduces the metastable zone width, so the crystals appear at a higher temperature. The effect is most dramatic in the case of leucine, where the metastable zone is decreased from about 22.5 K to about 9.8 K. In addition, the ultrasound has an effect on the crystal size distribution, so that the crystals are larger. For example Table 3 shows measurements of the resulting crystal size distributions, as measured with a Malvern Mastersizer 2000, for histadine and phenylalanine, showing the particle sizes (in µm) for different cumulative percentiles (by mass).

| TABLE 3 | 10% | 50% | 90% |
|---|---|---|---|
| Histadine no ultrasound | 15.8 | 59.9 | 166.3 |
| Histadine ultrasound | 20.6 | 89.6 | 370.8 |
| Phenylalanine no ultrasound | 133.9 | 352.4 | 655.9 |
| Phenylalanine ultrasound | 159.9 | 420.9 | 776.1 |

As another application, a saturated solution may be insonated so as to generate crystals, and then be added to a larger volume of solution so that the crystals act as seed crystals for the entire volume. For example there might be 4000 litres of a saturated solution in a crystallisation tank, which is gradually cooled or to which anti-solvent is added. When it is sufficiently supersaturated, a small quantity (eg 40 1) is transferred into an irradiation chamber (eg sucked up through a pipe) at the same temperature as the tank; there it is subjected to ultrasound so that crystals are formed; it is then transferred back into the tank. If no crystals are formed, this operation may be repeated.

## Claims

1. A method for production of crystalline material, the method comprising forming a saturated solution of the material, gradually changing the temperature of the solution so it becomes super-saturated, and subjecting the solution to irradiation by high-intensity ultrasound, wherein the method involves:
a) finding the temperature which provides the least degree of supersaturation at which crystals form from the solution on brief application of ultrasound, and then
b) subjecting the solution while it is supersaturated, at this temperature, to ultrasound for less than 5 seconds such that crystals are formed, and then
c) allowing the crystals in the solution to grow without irradiation by continuing to change the temperature of the solution,
wherein the ultrasound is provided to the supersaturated solution in a vessel using a multiplicity of ultrasonic transducers attached to a wall of the vessel in an array extending both circumferentially and longitudinally, each transducer being connected to a signal generator so that the transducer radiates no more than 3 W/cm², the transducers being sufficiently close together and the number of transducers being sufficiently high that the power dissipation within the vessel is between 25 and 150 W/litre.

2. A method as claimed in claim 1 wherein ultrasound is applied in such a way that no focusing of the ultrasound occurs.

3. A method as claimed in claim 2 wherein focusing is prevented by energising groups of adjacent transducers, in succession.

4. A method as claimed in claim 2 or claim 3 wherein focusing is prevented by energising adjacent transducers, or adjacent groups of transducers, at different frequencies.

5. A method as claimed in any one of the preceding claims wherein the crystalline material is aspartame.

6. A method as claimed in any one of the preceding claims wherein the crystalline material is an amino acid.

## Patentansprüche

1. Verfahren zur Erzeugung von kristallinem Material durch Bildung einer gesättigten Lösung des Materials, allmähliche Änderung der Temperatur der Lösung, damit sie übersättigt wird, und Aussetzen der Lösung einer Bestrahlung durch hochintensiven Ultraschall, wobei das Verfahren folgende Schritte umfasst:
a) Herausfinden der Temperatur, die den geringsten Grad von Übersättigung vorsieht, bei welcher sich Kristalle aus der Lösung durch kurze Anwendung von Ultraschall bilden; und dann
b) Aussetzen der Lösung, während sie bei dieser Temperatur übersättigt wird, dem Ultraschall für weniger als 5 Sekunden, so dass Kristalle gebildet werden; und dann
c) den Kristallen in der Lösung die Möglichkeit geben, ohne Bestrahlung durch fortgesetztes Ändern der Temperatur der Lösung zu wachsen,
wobei der Ultraschall der gesättigten Lösung in einem Behälter unter Verwendung einer Vielzahl von Ultraschallwandlern zugeführt wird, die an der Wand des Behälters in einer Anordnung angebracht sind, die sich sowohl in Umfangsrichtung als auch in Längsrichtung erstreckt, wobei jeder Wandler mit einem Signalgenerator verbunden ist, derart, dass der Wandler nicht mehr als 3W/cm² abstrahlt, wobei die Wandler genügend dicht beieinander angeordnet sind und die Anzahl von Wandlern genügend hoch ist, damit die Energiestreuung innerhalb des Behälters zwischen 25 und 150 W/Liter beträgt.

2. Verfahren nach Anspruch 1, bei dem der Ultraschall so zur Anwendung gebracht wird, dass keine Fokussierung des Ultraschalls stattfindet.

3. Verfahren nach Anspruch 2, bei dem eine Fokussierung durch Erregung von Gruppen benachbarter Wandler in Aufeinanderfolge verhindert wird.

4. Verfahren nach Anspruch 2 oder 3, bei dem eine Fokussierung durch Erregen benachbarter Wandler oder Gruppen von benachbarten Wandlern mit unterschiedlichen Frequenzen verhindert wird.

5. Verfahren nach einem der vorangehenden Ansprüche, bei dem das kristalline Material Aspartam ist.

6. Verfahren nach einem der vorangehenden Ansprüche, bei dem das kristalline Material eine Aminosäure ist.

## Revendications

1. Procédé de production d'une matière cristalline, le procédé comprenant la formation d'une solution saturée de la matière, le changement graduel de la température de la solution de façon à ce qu'elle devienne super-saturée, et la soumission de la solution à une exposition par des ultrasons haute intensité, dans lequel le procédé comprend :
a) la recherche de la température qui donne le moindre degré de super-saturation à laquelle des cristaux se forment à partir de la solution lors d'une brève application d'ultrasons, et ensuite
b) la soumission de la solution tandis qu'elle est super-saturée, à cette température, à des ultrasons pendant moins de 5 secondes de façon à ce que des cristaux soient formés, et ensuite
c) la croissance des cristaux dans la solution sans exposition en continuant le changement de la température de la solution,
dans lequel les ultrasons sont appliqués à la solution super-saturée dans une cuve en utilisant une multiplicité de transducteurs ultrasoniques fixés à une paroi de la cuve en un ensemble s'étendant à la fois circonférentiellement et longitudinalement, chaque transducteur étant connecté à un générateur de signaux de façon à ce que le transducteur ne projette pas plus de 3 W/cm², les transducteurs étant suffisamment proches les uns des autres et le nombre de transducteurs étant suffisamment élevé pour que la dissipation de puissance à l'intérieur de la cuve soit entre 25 et 150 W/litre.

2. Procédé selon la revendication 1 dans lequel les ultrasons sont appliqués de telle manière qu'aucune focalisation des ultrasons n'a lieu.

3. Procédé selon la revendication 2 dans lequel la focalisation est prévenue en mettant sous tension des groupes de transducteurs adjacents, en succession.

4. Procédé selon la revendication 2 ou la revendication 3 dans lequel la focalisation est prévenue en mettant sous tension des transducteurs adjacents, ou des groupes adjacents de transducteurs, à des fréquences différentes.

5. Procédé selon l'une quelconque des revendications précédentes dans lequel la matière cristalline est l'aspartame.

6. Procédé selon l'une quelconque des revendications précédentes dans lequel la matière cristalline est un acide aminé.
